# EUROPÄISCHE PATENTSCHRIFT

(11) **EP 0 513 600 B1**
(45) Veröffentlichungstag und Bekanntmachung des Hinweises auf die Patenterteilung: **11.01.1995**
(21) Anmeldenummer: 92107393.8
(22) Anmeldetag: 30.04.1992
(51) Int. Cl.: C07C 51/34

(54) **Verfahren zur Herstellung von Polycarbonsäuren und deren Derivate**
Process for preparing polycarboxylic acids and their derivatives
Procédé de préparation d'acides polycarboxyliques et de leurs dérivés

(30) Priorität: 02.05.1991 DE 4114376
(43) Veröffentlichungstag der Anmeldung: 19.11.1992
(73) Patentinhaber: HOECHST AKTIENGESELLSCHAFT, 65926 Frankfurt am Main (DE)
(72) Erfinder: Kulpe, Jürgen, Dr., W-6230 Frankfurt am Main 80 (DE); Strutz, Heinz, Dr., W-6230 Frankfurt am Main 80 (DE)

(56) Entgegenhaltungen:
- DE-B- 1 093 358
- US-A- 3 218 353
- US-A- 3 284 492

## Beschreibung

Die Erfindung betrifft ein Verfahren zur Herstellung von Polycarbonsäuren und deren Derivate, wobei die hergestellten Polycarbonsäuren mindestens drei Carboxylgruppen enthalten.
Die Erfindung betrifft insbesondere die Ozonolyse von organischen Verbindungen, die nicht aromatische C-C-Doppelbindungen enthatten und die oxidative Aufarbeitung der, bei der Oxidation dieser organischen Verbindungen, gebildeten Ozonide mit Wasserstoffperoxid zu Polycarbonsäuren.

Carbonsäuren und Polycarbonsäuren sind Ausgangsverbindungen für eine Vielzahl industrieller Anwendungen, wie z.B. die Herstellung von Polyestern. Neben solchen Anwendungen können typische Polycarbonsäuren, wie z.B. 1,2,3,4-Butantetracarbonsäure und 1,2,3,4-Cyclopentantetracarbonsäure als Cellulosevernetzer (Textile Research Journal **58** (8) 480 (1988)) eingesetzt werden.

Es sind zahlreiche Methoden zur Herstellung von Carbonsäuren und Polycarbonsäuren durch oxidative Spaltung nicht-aromatischer C-C-Doppelbindungen bekannt. Bei Verwendung von Cycloolefinen als Ausgangsstoffe zur oxidativen Spaltung von Kohlenstoff-Kohlenstoff-Doppelbindungen kann man unter geeigneten Reaktionsbedingungen ω,ω'-Dicarbonsäuren herstellen. Bei Verwendung von Norbornen-Derivaten mit einer Carboxylgruppe im bicyclischen Ringsystem, wie z.B. Norbornencarbonsäure, kann man unter geeigneten Reaktionsbedingungen Tricarbonsäuren herstellen. Bei Verwendung von Diels-Alder-Addukten wie z.B. dem Tetrahydrophthalsäureanhydrid, das durch Umsetzung von 1,3-Butadien als Dien mit Dienophilen wie Maleinsäureanhydrid, Maleinsäure oder Fumarsäure leicht herzustellen ist, kann man unter geeigneten Reaktionsbedingungen Tetracarbonsäuren, wie die 1,2,3,4-Butantetracarbonsäure herstellen.

Neben der Spaltung der C-C-Doppelbindung durch Permanganat, Wolframsäure oder Osmiumtetraoxid mit geeigneten Cooxidantien, ist die Spaltung der C-C-Doppelbindung durch Ozon eine seit langem bekannte Methode. Hierzu wird die olefinische Ausgangsverbindung in einem Lösungsmittel mit einem ozonhaltigen Trägergas, meist Sauerstoff, behandelt. Wird die Reaktion in einem aprotischen Lösungsmittel, wie z.B. Methylenchlorid, Estern, durchgeführt (Reaktion I), entstehen Sekundärozonide des Typs I, in protischen Lösungsmitteln, wie z.B. Alkoholen (R'-OH), Säuren, bilden sich Peroxide des Typs II, welche auch als Polymere vorliegen können, nach folgendem Schema: 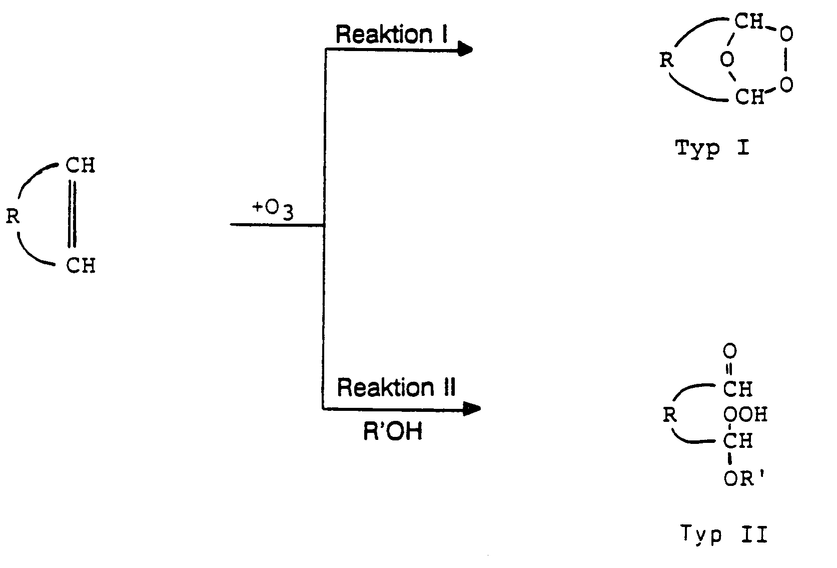
(R und R' sind sogenannte organische Reste z.B. Kohlenwassertstoffreste)
Im allgemeinen ist es günstig die Ozonolyse in protischen Lösungsmitteln durchzuführen, da es sich bei den Ozoniden des Typs I häufig um explosible Verbindungen handelt, die aufgrund ihrer geringen Löslichkeit aus dem Lösungsmittel ausfallen (siehe "Ozonization in Organic Chemistry", P. S. Bailey, Academic Press, New York/London, 1978). Setzt man die Ozonolyseprodukte des Typs I oder II mit Oxidationsmitteln um, erhält man die entsprechenden Dicarboxylverbindungen. Als Oxidationsmittel können Peroxocarbonsäuren z.B. Peroxoessigsäure, in situ Peroxosäuren z.B. Ameisensäure mit Wasserstoffperoxyd oder Essigsäure mit Wasserstoffperoxyd in Gegenwart einer katalytischen Menge einer starken Brönstedt-Säure wie Schwefelsäure, ebenso alkalische Wasserstoffperoxydlösung und auch Sauerstoff eingesetzt werden.

Beispielhaft für die Darstellung von Polycarbonsäuren soll die Darstellung von 1,2,3,4-Butantetracarbonsäure, im weiteren BTCA genannt, anhand einer Reihe von Publikationen und Patenten beschrieben werden.

W. S. Knowles et al. beschreiben in J. Org. Chem **30** (1965) 1488 die Herstellung von BTCA durch eine Vanadium-katalysierte Salpetersäureoxidation.

In EP-A-0 021 118 wird BTCA durch eine Ruthenium-katalysierte Koppeloxidation von Tetrahydrophthalsäureanhydrid und Acetaldehyd hergestellt. Dies hat einen Zwangsanfall der vielfachen molaren Menge an Essigsäure zur Folge.

EP-A-0 201 719 beschreibt die Oxidation mittels Wolframsäure und Wasserstoffperoxid. Nachteilig sind hier die hohen Katalysatorkosten und der benötigte Überschuß an Wasserstoffperoxid. Bei allen Metall-katalysierten Verfahren ergibt sich das Problem der Katalysatorentfernung. Je nach Verwendung der BTCA und Toxizität des Metalles ist eine Entfernung bis zur Nachweisgrenze notwendig.

Auch die Darstellung von BTCA durch Ozonolyse von Tetrahydrophthalsäure bzw. deren Anhydrid mit anschließender Oxidation der gebildeten Ozonide ist bekannt.

US-A-3 218 353 beschreibt ein Verfahren zur Herstellung von 1,2,3,4-Butantetracarbonsäure und 1,2,3,4-Cyclopentantetracarbonsäure durch Ozonolyse von Olefinen und anschließender oxidativer Zersetzung der gebildeten Ozonide in Lösungsmittel wie Methanol, Ethylacetat und Eisessig.

J. E. Franz, W. S. Knowles und C. Osuch in J. Org. Chem. **30** (1965) 4328 beschreiben ein Verfahren zur Herstellung von meso-1,2,3,4-Butantetracarbonsäure in Essigsäure und Ameisensäure, wobei die nachfolgende Oxidation der gebildeten Ozonide mit einer Peroxocarbonsäure durchgeführt wird, die entweder direkt zugegeben oder wie beschrieben in situ hergestellt wird. Die in situ-Methode bedingt in jedem Fall die Verwendung einer Säure als Lösungsmittel. Im Falle der in situ-Methode ist ein geringer Wassergehalt, d.h. die Verwendung von möglichst wasserfreier Säure als günstig angegeben, da der Wassergehalt in das Säure/Persäure-Gleichgewicht eingeht. Bei Verwendung von Essigsäure benötigt man zur Gleichgewichtseinstellung eine kleine Menge einer stärkeren Säure z.B. Schwefelsäure, welche unter Umständen nur schwer aus dem Produkt entfernt werden kann. Die ökologisch und ökonomisch notwendige Rückgewinnung der verwendeten Säure ist zumindest mit einem erheblichem technischen Aufwand verbunden. Im Fall der Ameisensäure ist eine vollständige Rückgewinnung sogar unmöglich, da sich diese bei Temperaturbelastung zersetzt. Verwendet man für die Ozonolyse statt einer Säure ein anderes Lösungsmittel wie z.B. Methanol, ist zur oxidativen Aufarbeitung ein Wechsel des Lösungsmittels notwendig. Dies ist ein erheblicher Nachteil, da zur destillativen Trennung Energie benötigt wird. Ferner birgt die Aufkonzentration der peroxidischen Reaktionsprodukte ein oft nicht unerhebliches Sicherheitsrisiko.

In US-A-3,284,492 und J. Org. Chem. **28** (1963) 2537 wird die Ozonolyse in wäßriger Emulsion beschrieben. Hierbei muß die wäßrige Phase Wasserstoffperoxid und Natriumhydroxid enthalten. Dadurch erhält man das Natriumsalz der entsprechenden Säure. In einem zweiten Reaktionsschritt wird dieses durch Zugabe von Salzsäure in die freie Carbonsäure überführt, was einen unerwünschten Zwangsanfall von Natriumchlorid zur Folge hat. Von Nachteil ist ebenfalls, daß das alkalische Milieu die Zersetzungsgeschwindigkeit des Wasserstoffperoxyds beschleunigt.

Es bestand daher die Aufgabe, ein Verfahren zur Herstellung von Polycarbonsäuren und deren Derivate zu entwickeln, das die oben angegebenen Nachteile umgeht.

Diese Aufgabe wird gelöst durch das Verfahren mit den in Anspruch 1 genannten Merkmalen. Die abhängigen Ansprüche geben besondere Ausführungsformen dieses Verfahrens an.

Bei dem erfindungsgemäßen Verfahren wird in Schritt (A) zunächst die organische Verbindung in das Reaktionsmedium Wasser eingebracht, wobei üblicherweise demineralisiertes Wasser verwendet wird. Dabei bestimmt die Qualität des verwendeten Wassers die Reinheit des hergestellten Endproduktes. Das erfindungsgemäße Verfahren zeichnet sich dadurch aus, daß die organische Verbindung in dem Reaktionsmedium nicht vollständig gelöst sein muß. Das erfindungsgemäße Verfahren läßt sich sowohl mit einer Lösung, einer Emulsion als auch mit einer Suspension der organischen Verbindung in Wasser durchführen. Voraussetzung zur Durchführung des erfindungsgemäßen Verfahrens ist lediglich eine teilweise Lösung der eingebrachten organischen Verbindung in dem Reaktionsmedium, wobei der Anteil der gelösten organischen Verbindung auch im Bereich der Nachweisgrenze liegen kann. Wasser stellt dabei in allen Schritten (A), (B) und (C) des erfindungsgemäßen Verfahrens sowohl Reaktionsmedium als auch Lösungsmittel dar.
Die Durchführung des erfindungsgemäßen Verfahrens mit einer Suspension der umzusetzenden Ausgangsverbindung ist überraschend, da die bekannten Verfahren zur Herstellung von Polycarbonsäuren den Einsatz in Lösung oder in Emulsion unter Verwendung eines Emulgators vorsehen.

In einem weiteren Schritt (B) des erfindungsgemäßen Verfahrens wird die Ozonolyse der organischen Verbindungen im Reaktionsmedium Wasser durchgeführt. Bei dem erfindungsgemäßen Verfahren wurde überraschender Weise gefunden, daß die Ozonolyse von organischen Verbindungen in Wasser durchführbar ist, gleichgültig in welcher Form diese organische Verbindungen in Wasser vorliegen, sei es als Lösung, Emulsion, oder sogar als Suspension in Wasser.

In Schritt (C) des erfindungsgemäßen Verfahrens erfolgt die Zugabe einer wässrigen Wasserstoffperoxidlösung. Dies führt zu einer oxidativen Aufarbeitung des nach der Ozonolyse vorhandenen Ozonids zur Polycarbonsäure. Dabei wurde überraschenderweise gefunden, daß die oxidative Aufarbeitung des Ozonolyseproduktes in Wasser ohne Zugabe einer Säure als Lösungsmittel durch einfache Zugabe von wäßriger Wasserstoffperoxydlösung erfolgen kann. Dies ist deshalb überraschend, da die Löslichkeit der freien Säure in Wasser erheblich geringer ist als die Löslichkeit des Natriumsalzes der Säure und die oxidative Aufarbeitung sogar in einer Suspension erfolgen kann. Die oxidative Aufarbeitung in einer Suspension gelingt erstaunlicherweise in sehr guten Ausbeuten, obwohl durch das Vorliegen einer Suspension nur ein kleiner Teil des gebildeten Ozonids der Reaktion zugänglich ist. Die Aufarbeitung in Wasser ist umso überraschender, da die oxidative Aufarbeitung mit Wasserstoffperoxid dem bisherigen Stand der Technik entsprechend nur unter Zusatz organischer Säuren wie Ameisensäure ohne weiteres gelingt. Schon bei der Verwendung von Essigsäure ist die Zugabe einer stärkeren Protonensäure wie Schwefelsäure nötig, um die, den Literaturangaben nach für die oxidative Aufarbeitung der Ozonolyseprodukte notwendige Persäure zu bilden. Das erfindungsgemäße Verfahren bietet die Möglichkeit die oxidative Aufarbeitung der Ozonolyseprodukte zu Polycarbonsäuren in wässriger Suspension ohne Zusatz einer organischen Säure oder eines Säuregemisches durchzuführen. Die erfolgreiche oxidative Aufarbeitung der Ozonolyseprodukte zu Polycarbonsäuren war insbesondere nicht zu erwarten, da die Reaktion in Wasser eine geringe Konzentration des Wasserstoffperoxyds mit sich bringt. Dennoch verläuft die oxidative Aufarbeitung der Ozonolyseprodukte in wässriger Lösung in mindestens vergleichbarer, teilweise sogar verbesserter Ausbeute im Vergleich zu den bisher bekannten Herstellungsverfahren ab.

Das erfindungsgemäße Verfahren läßt sich in allen Schritten (A), (B) und (C) auf eine große Zahl organischer Verbindungen anwenden. Diese organische Verbindungen können als Einzelverbindungen oder in Form von Gemischen mehrer Verbindungen in den Schritten (A), (B) und (C) eingesetzt werden, wobei die Anzahl der einzelnen Komponenten einer verwendeten Mischung zahlenmäßig nicht begrenzt sein muß. Unter organischen Verbindungen sind dabei Verbindungen zu verstehen, die nicht aromatische Kohlenstoff-Kohlenstoff-Doppelbindungen (C-C-Doppelbindungen) enthalten. Solche organischen Verbindungen, die ethylenische Doppelbindungen im Sinne von nicht aromatischen C-C-Doppelbindungen enthalten, werden im allgemeinen als Olefine bezeichnet und werden aus Gründen der Kürze desweiteren so bezeichnet. Dabei ist auch zu beachten, daß die Bezeichnung Olefine sich nicht auf Verbindungen beschränkt, die nur Kohlenstoff und Wasserstoff enthalten. Mit anderen Worten: Jede organische Verbindung (Olefin), enthaltend die nicht-aromatische C-C-Doppelbindungen, ist geeignet mittels des erfindungsgemäßen Verfahrens zu Polycarbonsäuren oder deren Derivate umgesetzt zu werden. Abhängig von der Struktur des Olefins sind die gebildeten Polycarbonsäuren mindestens Tricarbonsäuren. Im allgemeinen handelt es sich bei den verwendeten Olefinen um aliphatische Alkene, aliphatische Cycloalkene mit einer oder mehreren C-C-Doppelbindungen. Solche Olefine können in ihrer Struktur auch Substituenten mit einer aromatischen Gruppe, einer halogenhaltigen Gruppe, einer Nitro-, Carboxy-, Polycarboxy-, Alkoxy-, Aryloxy- oder Cyanogruppen enthalten.
Als Ausgangsstoffe lassen sich somit auch Ester und/oder Halbester mit nichtaromatischen C-C-Doppelbindungen einsetzen.
Kondensierte Ringsysteme in denen ein aromatisches Ringsystem mit einem Cycloalkenring verknüpft ist, wie z.B. 1,4-Dihydro-1,4-naphthalindicarbonsäure, können ebenfalls zur Herstellung von Polycarbonsäuren nach dem erfindungsgemäßen Verfahren eingesetzt werden.
Bicyclische Ringsysteme, wie z.B. Norbornendicarbonsäure, oder polycyclische Ringsysteme, wie z.B. Tetracyclo-[1.^{6.9}. 1.^{1.4}. 0.^{5.10}]-2,3-dicarboxy-7-decen, können ebenfalls als olefinische Ausgangsstoffe zur Herstellung der Polycarbonsäuren verwendet werden. Dabei können diese bicyclische oder polycyclischen Ringsysteme auch die eben erwähnten Gruppen und aromatischen Ringsysteme enthalten, ohne damit in ihrer Verwendung in dem erfindungsgemäßen Verfahren beschränkt zu sein.

Sogenannte Diels-Alder-Produkte, die durch Umsetzung eines Diens der allgemeinem Formel 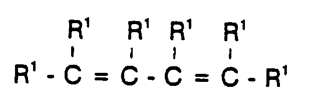
mit einem Dienophil, ausgewählt aus der Gruppe ungesättigter Dicarbonsäuren der allgemeinen Formel 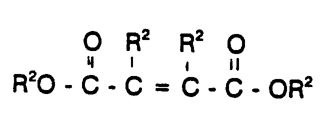
und Anhydride, Ester und Alkalisalze dieser Dicarbonsäuren, wobei R¹ und R² Wasserstoff, Alkyl- oder Arylreste darstellen, hergestellt wurden, sind ebenfalls geeignet als Olefine zur Herstellung von Polycarbonsäuren nach dem erfindungsgemäßen Verfahren eingesetzt zu werden.
Auch Diels-Alder-Produkte, die durch Umsetzung eines Diens der allgemeinen Formel 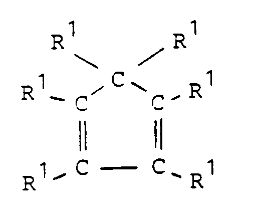
mit einem der obigen Dienophile hergestellt wurden, eignen sich ebenso zum Einsatz in dem erfindungsgemäßen Verfahren.

Zur Durchführung des erfindungsgemäßen Verfahrens wird zunächst in einem Schritt (A) die organische Verbindung in das Reaktionsmedium Wasser eingebracht. Das Reaktionsmedium Wasser in das die organische Verbindung eingebracht wird, besitzt einen pH-Wert von 7 oder kleiner 7. Dieses Einbringen der organischen Verbindung in das Reaktionsmedium kann auf verschiedene Weisen erfolgen. Das Einbringen kann auf die Weise erfolgen, daß das Reaktionsmedium Wasser in dem Reaktionsgefäß vorgelegt wird und die olefinische Ausgangsverbindung oder ein Gemisch mehrerer Ausgangsverbindungen zugegeben wird (diskontinuierliches Verfahren) oder das Reaktionsmedium Wasser und die olefinische Ausgangsverbindung oder ein Gemisch mehrerer Ausgangsverbindungen gleichzeitig in das Reaktionsgefäß gegeben werden (kontinuierliches Verfahren). Dabei können das Reaktionsmedium Wasser und die Ausgangsverbindung bzw. Gemisch mehrerer Ausgangsverbindungen zu gleichen oder unterschiedlichen Teilen in das Reaktionsgefäß gegeben werden. Die Durchführung eines solchen kontinuierlichen Verfahrens kann z. B. in einer Blasensäule erfolgen. Das Verhältnis von Wasser zu der organischen Verbindung wird nur durch die notwendige Durchmischung limitiert. Die notwendige Durchmischung wird durch eine geeignete Rührvorrichtung (z.B. Hochgeschwindigkeitsrührer) erreicht, die für eine gleichmäßige Verteilung der organischen Verbindung in dem Reaktionsmedium sorgt. Dabei kann es von Vorteil sein, die organische Verbindung in das bereits stark gerührte Reaktionsmedium zu geben. Die Mischung aus Reaktionsmedium und organischer Verbindung, sei es nun eine Lösung, Emulsion oder Suspension, wird nun für 15 bis 30 Minuten auf Temperaturen von 30°C bis Siedetemperatur, bevorzugt 80°C bis Siedetemperatur, erwärmt. Hierdurch entsteht aus einem eingesetzten Anhydrid die freie Carbonsäure, welche im Vergleich zum entsprechenden Anhydrid in höherer Konzentration in Wasser löslich ist, was für die Reaktion günstig ist. Dies schließt jedoch nicht aus, daß die Reaktion auch direkt mit dem Anhydrid durchgeführt werden kann. Will man teilveresterte Polycarbonsäuren herstellen, kann auch der Einsatz von Ester oder Halbester der entsprechenden organischen Ausgangsverbindung sinnvoll sein. Als Alkohol-Komponente können Mono- oder Polyhydroxy-Verbindungen mit einem Gerüst von 1 bis 10 Kohlenstoffatomen verwendet werden.

In Schritt (B) des erfindungsgemäßen Verfahrens wird Ozon in die Lösung, Emulsion oder Suspension eingeleitet, wobei zur besseren Begasung des Reaktionsansatzes der Rührvorgang nicht unterbrochen werden soll. Ozon kann in unterstöchiometrischer bis stark überstöchiometrischer Menge eingesetzt werden. Es werden bevorzugt 0,5 bis 10, besonders bevorzugt 1,0 bis 1,2 Äquivalente Ozon pro Mol nicht aromatische Doppelbindung der Ausgangsverbindung(en) eingesetzt.

Das Ozon kann in einem Trägergas z.B. Sauerstoff, Luft enthalten sein, welches seinerseits keine nachteiligen Auswirkungen auf die Reaktion hat oder in einem inerten Trägergas wie z.B. Stickstoff. Die Konzentration an Ozon in dem sich ergebenden ozonhaltigen Trägergasstrom ist nicht kritisch und erstreckt sich über weite Bereiche. Das Ozon kann in einem Trägergas bevorzugt in einer Konzentration von 0,1 bis 30 Vol.-%, besonders bevorzugt 1 bis 9 Vol.-% enthalten sein. In den Fällen in denen der Gasstrom einen geringen Gehalt an Ozon enthält, wird dieser in einem größeren Volumen und über einen längeren Zeitraum in die gerührte Reaktionsmischung eingeleitet. Ist die Konzentration des Ozons in dem Trägergas höher, werden kleinere Volumina an Trägergas in die gerührte Reaktionsmischung eingeleitet. Während der Einleitung sollte die Reaktionsmischung, bestehend aus dem Reaktionsmedium Wasser und dem eingesetzten Olefin, eine Temperatur zwischen dem Festpunkt der Lösung und dem Siedepunkt der Lösung haben, bevorzugt wird eine Temperatur von 0 bis 30°C, besonders bevorzugt eine Temperatur bei 5 bis 15°C. Dabei hat sich überaschender Weise gezeigt, daß die Ozonolyse gerade bei verhältnismäßig niedriger Temperatur den besten Reaktionsverlauf zeigt, obwohl die Löslichkeit des eingesetzten Olefins mit abnehmender Temperatur geringer wird. Die Anströmgeschwindigkeit des Ozon/Trägergasgemisches ist nicht kritisch. Sie wird vorteilhaft in Abhängigkeit der Reaktionstemperatur so gewählt, daß ein möglichst vollständiger Umsatz stattfindet und keine oder nur eine geringe Menge Ozon aus dem Reaktionsgefäß entweicht. Dies kann bedeuten, daß gegen Ende die Anströmgeschwindigkeit und/oder die Ozonkonzentration reduziert wird.

In Schritt (C) des erfindungsgemäßen Verfahrens erfolgt die oxidative Aufarbeitung der Reaktionsmischung. Zu diesem Zweck wird Wasserstoffperoxydlösung in den Reaktionsansatz gegeben. Es kann handelsübliches Wasserstoffperoxid verwendet werden. Die Wasserstoffperoxid-Konzentration der zugegebenen Lösung ist in weiten Bereichen variierbar; bevorzugt wird eine Konzentration von 10 bis 90 Gew.-%, insbesondere eine Konzentration von 50 bis 85 Gew.-% an Wasserstoffperoxid. Die verwendete Menge an Wasserstoffperoxid sollte von 0,1 bis 10 Äquivalenten, bevorzugt von 0,5 bis 2 Äquivalenten, besonders bevorzugt von 1,0 bis 1,75 Äquivalenten pro Mol nicht aromatische Doppelbindung der eingesetzten Ausgangsverbindung(en) betragen. Das Wasserstoffperoxid kann auch vor der Ozonolyse in die Reaktionslösung gegeben werden. Ebenso ist es möglich mit dem Wasserstoffperoxid eine katalytische Menge einer starken Säure wie z.B. Ameisensäure zuzugeben, was je nach Aufarbeitungstemperatur von Vorteil sein kann. Überraschenderweise hat sich jedoch gezeigt, daß die oxidative Aufarbeitung des Ozonolyseproduktes in dem Reaktionsmedium Wasser ohne Zugabe einer Säure als zusätzliches Lösungsmittel und/oder organischen Lösungsmittel erfolgen kann. Noch viel überraschender ist, daß die oxidative Aufarbeitung des Ozonolyseproduktes ohne Säurezugabe in mindestens vergleichbarer, teilweise sogar besserer Ausbeute erfolgt. Die Reaktionslösung wird nun 0,1 bis 70 Stunden bevorzugt 3 bis 30 Stunden auf eine Temperatur von 20°C, bevorzugt 40°C bis Siedetemperatur der Lösung, insbesondere 60 bis 80°C erwärmt. Im Bedarfsfall wird ein eventuell vorhandener Peroxidüberschuß durch Zugabe einer die Zersetzung katalysierenden Verbindung wie z.B. Aktivkohle und/oder Erwärmen zum Siedepunkt der Lösung zerstört. Gegebenenfalls nach einer Filtration wird das Wasser entfernt. Dies kann durch Abdestillation unter reduziertem Druck, z.B. in einem Rotationsverdampfer, oder beispielsweise durch Sprühtrocknung geschehen. Hierbei ist wiederum günstig, daß durch die Verwendung von Wasser als Reaktionsmedium auf die unter Umständen aufwendige, mit zusätzlichen Sicherheitsmaßnahmen behaftete Rückgewinnung des Lösungsmittels verzichtet werden kann.

In dem erhaltenen Rückstand kann der Gehalt an Polycarbonsäure sowohl durch quantitative Gaschromatographie nach Silylierung als auch durch Flüssigkeitschromatographie (HPLC) bestimmt werden. Die je nach Reaktionsbedingungen in sehr guten Ausbeuten erhaltene Polycarbonsäure ist für die meisten Anwendungen ohne weitere Reinigungsoperationen einsetzbar. Eine eventuell erforderliche Reinigung kann beispielsweise durch Umkristallisation erfolgen.
Werden durch das erfindungsgemäße Verfahren Halbester von Polycarbonsäuren hergestellt, kann ihr Gehalt in dem erhaltenen Rückstand ebenfalls durch quantitative Gaschromatographie und durch Flüssigkeitschromatographie (HPLC) bestimmt werden.
Die quantitative Bestimmung der hergestellten Ester der Polycarbonsäuren erfolgt mittels Gaschromatographie. Neben den gerade beschriebenen analytischen Methoden zur Bestimmung des Gehaltes an Endprodukt eignen sich alle Methoden, die zur Bestimmung des Carboxylgehaltes einer organischen Verbindung verwendet werden, wie z.B. Säure-Base-Titration oder Bestimmung der Säurezahl.

Das beschriebene Verfahren erlaubt die Herstellung von Polycarbonsäuren und deren Derivate in sehr hohen Ausbeuten. Das erfindungsgemäße Verfahren besteht aus mehreren Schritten (A), (B) und (C), wobei es hinsichtlich der Durchführung des Verfahrens möglich ist, daß die Zugabe des Wasserstoffperoxids (C) sowohl vor, während als auch nach der Ozonolyse der Ausgangsverbindungen (B) erfolgen kann. Die Folge der einzelnen Reaktionsschritte kann lauten: (A) dann (B) dann (C) oder (A) dann (C) dann (B) oder (A) dann (B) und (C).

Das erfindungsgemäße Verfahren hat gegenüber dem Stand der Technik erhebliche ökonomische und ökologische Vorteile, da zu keinem Zeitpunkt die Verwendung eines organischen Lösungsmittels zwingend notwendig ist. Durch die Reaktionsführung in Wasser entfällt eine aufwendige Rückgewinnung bzw. Entsorgung des Lösungsmittels. Die eingesetzten Oxidationsmittel Ozon und Wasserstoffperoxid ergeben als Endprodukte Sauerstoff und Wasser. Aus der Herstellung ergeben sich keine salzartigen Abfälle. Metall-Katalysatoren werden in der Reaktion nicht eingesetzt. Schwermetallkontaminationen sind somit nicht möglich, wodurch eine aufwendige Reinigung und Analytik entfällt.

Das erfindungsgemäße Verfahren wird anhand der nachfolgenden Beispiele und Vergleichsbeispiele näher erläutert, was nicht so zu verstehen ist, daß das erfindungsgemäße Verfahren allein auf diese Beispiele beschränkt ist.

In der erläuternden Ausführung des erfindungsgemäßen Verfahrens, die durch die nachfolgend angeführten Beispiele gegeben ist, bestand das Reaktionsgefäß aus einem 2l-Glasmehrhalskolben, versehen mit Hochgeschwindigkeitsrührer, Gaseinleitungsrohr mit Gasfritte, Gasentlüftung und Thermometer

### Beispiel 1

In einem 2 l Kolben werden 76,08 g (0,5 mol) Tetrahydrophthalsäureanhydrid in 600 ml Wasser 1 Stunde unter Rückfluß zum Sieden erhitzt. Nach dem Abkühlen leitet man Sauerstoff mit einem Ozongehalt von 2,9 Vol-% bei einer Strömungsgeschwindigkeit von 100 l h⁻¹ durch eine Fritte in die kräftig gerührte Suspension über einen Zeitraum von 3 Stunden und 30 Minuten ein. Dabei hält man die Temperatur der Lösung von 20 bis 25°C. 10 Minuten vor Ende der Reaktion unterbricht man die Einleitung kurzzeitig, um eventuell an der Wand des Gefäßes hängenden Feststoff herunter zu spülen. Am Ende der Reaktion hat man 714 ml einer klaren Lösung mit einer Dichte von 1,056 g ml⁻¹. Die Lösung wird halbiert.
- a:: 375 ml der Reaktionslösung werden mit 20 ml 60 gew.-prozentigem Wasserstoffperoxyd (437 mmol) 15 Stunden auf 70 bis 80°C erwärmt. Anschließend erhitzt man zum Rückfluß und gibt 500 mg gepulverte Aktivkohle zu. Wenn der Test auf Oxidationsmittel mit Kl-Stärkepapier negativ ausfällt, wird die Aktivkohle abfiltriert. Die erhaltene Lösung wird im Ölpumpenvakuum zur Trockne eingeengt. Der Rückstand wird bei 60°C bis zur Gewichtskonstanz getrocknet. Man erhält 56,05 g eines feinkristallinen Pulvers, dies entspricht 95,7 % der Theorie. Die Substanz hat einen Schmelzpunkt von 181 bis 186°C. Der Wassergehalt nach Karl-Fischer beträgt 4,2 Gew.-%, der Gehalt an Butantetracarbonsäure (BTCA) beträgt 93,4 Gew.-%. Dies ergibt eine Ausbeute von 89,4 % BTCA.
- b:: Die verbleibenden 375 ml Reaktionslösung werden mit 0,1 ml Ameisensäure versetzt. Anschließend wird wie unter a: verfahren. Man erhält 55,36 g Feststoff, was 94,6 % der Theorie entspricht. Dieser hat einen Gehalt von 88,9 Gew.-% BTCA, womit sich eine Ausbeute von 84,1 % BTCA ergibt.

### Beispiel 2

Der Ansatz entspricht dem Beispiel 1, jedoch werden 106,52 g (0,7 mol) Tetrahydrophthalsäureanhydrid eingesetzt. Die Gaseinleitung dauert 6 Stunden und 30 Minuten. Man erhält 670 ml Reaktionslösung mit einer Dichte von 1,084 g cm⁻³. Der Ansatz wird halbiert.
Zu 335 ml Reaktionslösung gibt man 19 ml 60 gew.-prozentigem Wasserstoffperoxyd (416 mmol) und erwärmt 24 Stunden auf 70°C, gibt 1 g gepulverte Aktivkohle zu und erhitzt unter Rückfluß 4 Stunden zum Sieden. Man erhält 78 g eines Feststoffes dies entsprechend 95,2 % der Theorie. Die Substanz hat einen Schmelzpunkt von 183 bis 186°C. Aus quantitativem GC und HPLC ergibt sich ein Gehalt von 95,2 Gew.-% an BTCA, woraus eine Ausbeute von 90 % folgt.

Mit der zweiten Hälfte wird analog verfahren, jedoch erhitzt man nach der Waserstoffperoxyd-Zugabe für 4 Stunden unter Rückfluß zum Sieden erhitzt. Man erhält eine Auswaage von 75,13 g eines weißen Pulvers, 91,7 % der Theorie. Die Substanz hat einen Schmelzpunkt von 183 bis 186°C. Aus einem Gehalt von 88,9 Gew.-% BTCA ergibt sich eine Ausbeute von 81,5 %.

### Beispiel 3

Der Ansatz entspricht Beispiel 2, jedoch wird dieser nicht geteilt und das Wasserstoffperoxyd (38 ml; 60 gew.-%ig; 832 mmol) wird vor der Ozonolyse zugegeben. Im Anschluß an die Ozonolyse wird 14 h auf 70°C erwärmt.
Auswaage: 153,54 g eines weißen Pulvers gleich 93,7 % der Theorie. Die Substanz hat einen Schmelzpunkt von 183 bis 184°C.

### Beispiel 4

164,16 g (1 mol) Norbornen-2,3-dicarbonsäureanhydrid werden in 500 ml Wasser 1 Stunde unter Rückfluß zum Sieden erhitzt. Beim Abkühlen auf Raumtemperatur fällt der größte Teil der freien Säure wieder aus. In die erhaltene Suspension leitet man bei einer Innentemperatur von 0 bis 10°C innerhalb 4 Stunden 1,1 Mol Ozon ein. Die Strömungsgeschwindigkeit wird hierbei zwischen 100 und 200 l h⁻¹ variiert. Anschließend werden 32,5 ml 85 gew.-prozentiges Wasserstoffperoxyd (1,1 mol) zugegeben und zunächst 12 Stunden auf 75°C, dann 3 Stunden zur Siedehitze erwärmt. Nach der Zugabe von 1 g gepulverter Aktivkohle wird weiter erhitzt, bis der Test auf Oxidationsmittel mit KI-Stärkepapier negativ ausfällt. Nach der Filtration der Aktivkohle wird das Lösungsmittel am Rotationsverdampfer abgezogen und der erhaltene Rückstand bis zur Gewichtskonstanz getrocknet. Man erhält 241 g eines weißen Feststoffes, dies entspricht 98 % der Theorie. Dieser wird durch 1H-NMR-Spektroskopie sowie Gaschromatographie mit massenselektiver Detektion als Cyclopentantetracarbonsäure identifiziert.

### Beispiel 5

In eine Emulsion von 12 ml (99,6 mmol) (2-Cyclopenten-1-yl)essigsäure und 30 ml Wasser werden bei 0 bis 5°C innerhalb 30 Minuten 110 mmol Ozon eingeleitet. In Anschluß werden 3,75 ml 85 gew.-prozentiges Wasserstoffperoxyd (125 mmol) zugegeben und die Temperatur 12 h auf 70°C erhöht. Anschließend erhitzt man unter Rückfluß zum Sieden und gibt 250 mg gepulverte Aktivkohle zu. Wenn ein Test auf Oxidationsmittel mit KI-Stärkepapier negativ ausfällt, wird die Aktivkohle abfiltriert und das Lösungsmittel abgezogen. Der Rückstand wird bis zur Gewichtskonstanz getrocknet.

Die Auswaage ergibt 15,4 g gelbliches Pulver, was 81 % der Theorie entspricht. Durch 1H-NMR-Spektroskopie sowie Gaschromatographie mit massenselektiver Detektion wird dieses als 1,2,4-Butantricarbonsäure identifiziert.

## Patentansprüche

1. Verfahren zur Herstellung von Polycarbonsäuren mit mindestens drei Carboxylgruppen und deren Derivate, umfassend die Schritte
(A) Einbringen einer oder mehrerer organischer Verbindungen, die nicht aromatische Kohlenstoff-Kohlenstoff (C-C) -Doppelbindungen enthalten in Wasser,
(B) Ozonolyse der organischen Verbindungen in Wasser durch Einleiten eines ozonhaltigen Trägergases,
(C) Zusatz von wäßriger Wasserstoffperoxidlösung, um die in Schritt (B) entstandenen Ozonolyseprodukte oxidativ aufzuarbeiten, dadurch gekennzeichnet, daß im Schritt (A) die organische(n) Verbindung(en) in Wasser mit einem pH-Wert von 7 oder kleiner 7 eingebracht wird (werden) und daß der Zusatz von Wasserstoffperoxid im Schritt (C) in Wasser ohne Zusatz eines organischen Lösungsmittels erfolgt.

2. Verfahren nach Anspruch 1, dadurch gekennzeichnet, daß die organische(n) Verbindung(en) in wäßriger Lösung oder Emulsion, bevorzugt in wäßriger Suspension eingesetzt wird (werden).

3. Verfahren nach Anspruch 1 oder 2, dadurch gekennzeichnet, daß als organische Verbindung(en) Tetrahydrophthalsäure und/ oder Norbornendicarbonsäure eingesetzt wird (werden).

4. Verfahren nach einem der Ansprüche 1 bis 3, dadurch gekennzeichnet, daß im Schritt (B) 0,5 bis 10, besonders bevorzugt 1,0 bis 1,2 Äquivalente Ozon pro Mol nicht aromatische Doppelbindung der Ausgangsverbindung(en) eingesetzt werden.

5. Verfahren nach einem der Ansprüche 1 bis 4, dadurch gekennzeichnet, daß das Ozon in einem Trägergas in einer Konzentration von 0,1 bis 30 Gew.-%, bevorzugt 1 bis 9 Gew.-% enthalten ist.

6. Verfahren nach einem der Ansprüche 1 bis 5, dadurch gekennzeichnet, daß die Ozonolyse bei einer Temperatur von maximal dem Siedepunkt der Lösung, bevorzugt bei 0 bis 30°C, insbesondere bei 5 bis 15°C durchgeführt wird.

7. Verfahren nach einem der Ansprüche 1 bis 6, dadurch gekennzeichnet, daß die verwendete Menge an Wasserstoffperoxid von 0,1 bis 10 Äquivalenten, bevorzugt 0,5 bis 2 Äquivalenten, besonders bevorzugt 1,0 bis 1,75 Äquivalenten pro Mol nicht aromatische Doppelbindung der eingesetzten Ausgangsverbindung(en) beträgt.

8. Verfahren nach einem der Ansprüche 1 bis 7, gekennzeichnet, durch die Folge der Reaktionsschritte (A) dann (B) dann (C) oder (A) dann (C) dann (B) oder (A) dann (B) und (C).

9. Verfahren nach einem der Ansprüche 1 bis 8, dadurch gekennzeichnet, daß Wasserstoffperoxid während der Ozonolyse zugegeben wird.

10. Verfahren nach einem der Ansprüche 1 bis 9, dadurch gekennzeichnet, daß die oxidative Aufarbeitung des Ozonolyseproduktes im Schritt (C) in Anwesenheit einer katalytischen Menge einer Säure durchgeführt wird.

11. Verfahren nach einem der Ansprüche 1 bis 10, dadurch gekennzeichnet, daß man die Reaktionslösung zur oxidativen Aufarbeitung der Ozonolyseprodukte auf eine Temperatur von mindestens 20°C, bevorzugt 40°C, bis Siedetemperatur der Lösung, insbesondere bei 60 bis 80°C erwärmt.

## Claims

1. A process for the preparation of a polycarboxylic acid having at least three carboxyl groups and derivatives thereof, comprising the steps
(A) introducing one or more organic compounds, which contain nonaromatic carbon-carbon (C-C) double bonds, into water,
(B) ozonolysis of the organic compounds in water by passing in an ozone-containing carrier gas,
(C) addition of an aqueous hydrogen peroxide solution, to oxidatively work up the ozonolysis products produced in step (B),
which comprises, in step (A), introducing the organic compound(s) into water having a pH of 7 or less than 7 and, in step (C), adding hydrogen peroxide in water without adding an organic solvent.

2. The process as claimed in claim 1, wherein the organic compound(s) is(are) used in aqueous solution or emulsion, preferably in aqueous suspension.

3. The process as claimed in claim 1 or 2, wherein the organic compound(s) used is(are) tetrahydrophthalic acid and/or norbornenedicarboxylic acid.

4. The process as claimed in one of claims 1 to 3, wherein, in step (B), 0.5 to 10, particularly preferably 1.0 to 1.2, equivalents of ozone are used per mole of nonaromatic double bond of the starting compound(s).

5. The process as claimed in one of claims 1 to 4, wherein the ozone is contained in a carrier gas at a concentration of 0.1 to 30% by weight, preferably 1 to 9% by weight.

6. The process as claimed in one of claims 1 to 5, wherein the ozonolysis is carried out at a temperature of at most the boiling point of the solution, preferably at 0 to 30°C, in particular at 5 to 15°C.

7. The process as claimed in one of claims 1 to 6, wherein the amount of hydrogen peroxide used is 0.1 to 10 equivalents, preferably 0.5 to 2 equivalents, particularly preferably 1.0 to 1.75 equivalents per mole of nonaromatic double bond of the starting compound(s) used.

8. The process as claimed in one of claims 1 to 7, wherein the sequence of the reaction steps is (A) then (B) then (C) or (A) then (C) then (B) or (A) then (B) and (C).

9. The process as claimed in one of claims 1 to 8, wherein hydrogen peroxide is added during the ozonolysis.

10. The process as claimed in one of claims 1 to 9, wherein the oxidative work-up of the ozonolysis product in step (C) is carried out in the presence of a catalytic amount of an acid.

11. The process as claimed in one of claims 1 to 10, wherein the reaction solution is heated for the oxidative work-up of the ozonolysis products to a temperature of at least 20°C, preferably 40°C, up to the boiling temperature of the solution, in particular at 60 to 80°C.

## Revendications

1. Procédé de préparation d'acides polycarboxyliques avec au moins trois groupes carboxyle et de leurs dérivés, comprenant les étapes :
(A) introduction d'un ou plusieurs composés organiques qui contiennent des doubles liaisons carbone-carbone (C-C) non aromatiques dans de l'eau,
(B) ozonolyse des composés organiques dans l'eau par introduction d'un gaz porteur contenant de l'ozone,
(C) addition de solution de peroxyde d'hydrogène aqueuse pour traiter de façon oxydative les produits d'ozonolyse résultant de l'étape (B), caractérisé en ce que dans l'étape (A) le (les) composé(s) organique(s) est (sont) introduit(s) dans l'eau avec une valeur de pH de 7 ou inférieure à 7 et en ce que l'addition de peroxyde d'hydrogène dans l'étape (C) dans l'eau a lieu sans addition d'un solvant organique.

2. Procédé selon la revendication 1, caractérisé en ce que le (les) composé(s) organique(s) est (sont) mis en oeuvre en solution ou émulsion aqueuse, de préférence en suspension aqueuse.

3. Procédé selon la revendication 1 ou 2, caractérisé en ce qu'on met en oeuvre, en tant que composé(s) organique(s), de l'acide tétrahydrophtalique et/ou de l'acide norbornène dicarboxylique.

4. Procédé selon l'une des revendications 1 à 3, caractérisé en ce que dans l'étape (B) on utilise 0,5 à 10, de façon particulièrement préférée 1,0 à 1,2 équivalent d'ozone par mol de double liaison non aromatique du (des) composé(s) de départ.

5. Procédé selon l'une des revendications 1 à 4, caractérisé en ce que l'ozone est contenu dans un gaz porteur à une concentration de 0,1 à 30 % en poids, de préférence de 1 à 9 % en poids.

6. Procédé selon l'une des revendications 1 à 5, caractérisé en ce que l'ozonolyse est effectuée à une température au maximum égale à la température d'ébullition de la solution, de préférence à une température de 0 à 30°C, en particulier à une température de 5 à 15°C.

7. Procédé selon l'une des revendications 1 à 6, caractérisé en ce que la quantité de peroxyde d'hydrogène utilisée est de 0,1 à 10 équivalents, de préférence de 0,5 à 2 équivalents, de façon particulièrement préférée de 1,0 à 1,75 équivalent par mol de double liaison non aromatique du (des) produit(s) de départ mis en oeuvre.

8. Procédé selon l'une des revendications 1 à 7, caractérisé en ce que la suite des étapes de réaction est (A) puis (B) puis (C) ou (A) puis (C) puis (B) ou (A) puis (B) et (C).

9. Procédé selon l'une des revendications 1 à 8, caractérisé en ce que le peroxyde d'hydrogène est ajouté pendant l'ozonolyse.

10. Procédé selon l'une des revendications 1 à 9, caractérisé en ce que le traitement oxydatif du produit d'ozonolyse dans l'étape (C) est effectué en présence d'une quantité catalytique d'un acide.

11. Procédé selon l'une des revendications 1 à 10, caractérisé en ce qu'on chauffe la solution réactionnelle pour le traitement oxydatif des produits d'ozonolyse à une température d'au moins 20°C, de préférence 40°C, jusqu'à la température d'ébullition de la solution, en particulier à une température de 60 à 80°C.
